Europäisches Patentamt

European Patent Office

Office européen des brevets

(11) Publication number: **0 100 616**
A2

# (12) EUROPEAN PATENT APPLICATION

(21) Application number: **83304048.8**

(51) Int. Cl.³: **A 61 K 7/16**

(22) Date of filing: **12.07.83**

(30) Priority: **15.07.82 US 398343**

(43) Date of publication of application:
**15.02.84 Bulletin 84/7**

(84) Designated Contracting States:
**AT BE CH DE FR GB LI NL**

(71) Applicant: **Amway Corporation**
**7575 East Fulton Road**
**Ada Michigan 49355(US)**

(72) Inventor: **Strobridge, John Roger**
**1295 Buth N.E.**
**Comstock Park Michigan 49321(US)**

(74) Representative: **Perry, Robert Edward et al,**
**GILL JENNINGS & EVERY 53-64 Chancery Lane**
**London WC2A 1HN(GB)**

(54) Fluoride toothpastes.

(57) A fluoride toothpaste comprises a kaolin abrasive which is at least 80% calcined and which has a particle size distribution such that the majority of particles are between 1 and 10 microns, and not more than 35% are under 1 micron, in diameter. The resulting toothpaste shows surprising efficacy in terms of fluoride availability, fluoride uptake on human enamel, enamel solubility reduction and polishing and stain removal.

EP 0 100 616 A2

AMWAY                              1              GJE 70/2166/02

## FLUORIDE TOOTHPASTES

The present invention relates to fluoride toothpastes using kaolin as the abrasive ingredient. Kaolin is known to be a desirable abrasive for toothpastes because it is inexpensive and because it cleans and polishes effectively.

US-A-4,122,163 discloses a fluoride toothpaste using calcined kaolin as the abrasive. The kaolin used is said to be predominantly of the gamma-alumina or mullite form. As kaolin is calcined, it undergoes a fairly sharp endothermic reaction at about 520°C, where it converts to the gamma-alumina crystal form. As heating continues, the kaolin undergoes an exothermic reaction at about 975-990°C, where the crystal form changes from the gamma-alumina to the mullite form.

Although the gamma-alumina/mullite kaolin disclosed in US-A-4,122,163 has a very high degree of purity, having a whiteness index of not more than 10 and a brightness of at least about 93.0, it has been found, surprisingly, that it exerts a detrimental affect on fluoride availability as the toothpaste ages. An adverse impact on fluoride availability might have been expected if there were impurities in the kaolin. In this context and throughout the specification, fluoride availability refers to the availability of soluble fluoride ion in the toothpaste composition. To the extent that the fluoride reacts or complexes with other ingredients in the toothpaste, it becomes effectively unavailable for the purpose for which it is intended, i.e. to be taken up by the enamel of the teeth.

Particle size is important to polishing efficacy. It is well-known that larger-sized particles, for example, those in excess of about 10 microns, must be avoided in order to avoid excessive abrasion of the teeth. Finer particles are known to afford better polishing results without abrasion. US-A-4,122,163 emphasises that the particles must be predominantly less than about 10 microns in diameter, with a major portion being even less than 2 microns in diameter. JP-B-24,224 of 1974 emphasises that all of the kaolin particles should be less than 1 micron in diameter.

US-A-3,105,013 and 3,282,792 disclose the use of Kaotolite SF as an abrasive. This material is highly calcined, with 65% of the abrasive particles being less than 1 micron in size.

While it would be desirable to use kaolin as an abrasive in fluoride toothpastes, no such toothpastes appear ever to have been commercialised.

It has been discovered that, if there is an excessive amount of particles under 1 micron, the product rheology is adversely affected. It becomes too stringy. This is undesirable both from the aesthetic viewpoint of the product user, and from a processing standpoint. The stringiness impedes processing of the product during manufacture. It was then found that particle size can have an adverse effect on fluoride availability.

According to the present invention, a fluoride toothpaste comprises calcined kaolin which is at least 80% calcined to the mullite crystal form; the kaolin must also have a particle size distribution such that the majority of the particles are sufficiently fine to afford good polishing without abrasion, but such that less than 35% of those particles are under 1 micron in diameter.

A fluoride toothpaste of the invention shows not only a high degree of fluoride availability when the product is first formulated, but also shows a significant resistance to reduction in fluoride availability with aging. The product also exhibits good polishing properties and good *in vitro* fluoride uptake by human enamel.

The kaolin used in a fluoride toothpaste of the present invention is at least 80%, and preferably at least 90%, calcined to the mullite crystalline form. It preferably has a particle size distribution such that more than 50% of the particles lie between 1 and 10, and preferably between 1 to 5 microns, and that less than 5% exceed 10 microns, while less than 35%, more preferably less than 30%, are less·than 1 micron, in diameter. Most preferably, no more than 10% are from 5 to 10 microns in size.

A preferred formula for a toothpaste of the invention comprises from 5 to 70% of kaolin as defined, from 0.05 to 1% fluoride ion (although 0.1% fluoride ion is the maximum allowed by the US Food and Drug Administration (F.D.A.) in an over-the-counter fluoride toothpaste), from 5 to 70% homectant and from 0.5 to 10% binder. Other desirable ingredients include from 0.5 to 5% surfactant, flavouring ingredients, 0.05 to 10% titanium dioxide whitener, sequestering agents and preservatives. All percentages are by weight.

The following Table I gives suitable ranges for preferred constituents of a toothpaste of the invention, and also gives a specific example, of preferred amounts of those components for a single toothpaste formulation.

TABLE I

| | % w/w | range (% w/w) | | |
|---|---|---|---|---|
| Kaolin as defined herein | 37.0000 | 5.0 | – | 70.0 |
| Deionised Water | 24.0006 | | q.s. | |
| Glycerin | 14.0000 | 5.0 | – | 70.0 |
| Sorbitol | 17.5000 | 5.0 | – | 70.0 |
| Titanium Dioxide | 2.0000 | 0.05 | – | 10.0 |
| Carboxymethylcellulose | 1.3500 | 0.50 | – | 5.0 |
| Sodium Lauryl Sulfate | 1.5000 | 0.50 | – | 5.0 |
| Flavouring Agent | 0.8500 | 0.05 | – | 5.0 |
| Magnesium Aluminium Silicate | 0.6230 | 0.05 | – | 5.0 |
| Sodium Saccharin | 0.3000 | 0.05 | – | 5.0 |
| Sodium Citrate | 0.2500 | 0.05 | – | 5.0 |
| Sodium Phosphate | 0.2000 | 0.05 | – | 5.0 |
| Methyl Paraben | 0.1500 | 0.01 | – | 5.0 |
| Propyl Paraben | 0.0500 | 0.005 | – | 5.0 |
| Colouring Agent | 0.0020 | 0.00001 | – | 2.00 |
| Sodium fluoride | 0.2244 | 0.05 | – | 1.0 |

The glycerin and sorbitol act as humectants, and also serve as sweeteners. Titanium dioxide is employed to give the toothpaste a bright white appearance. Carboxymethylcellulose and magnesium aluminium silicate are both binders. Sodium lauryl sulfate is added as a surfactant. Sodium saccharin is the primary sweetener. Sodium citrate is added as a sequestering agent to tie up any free trivalent ions, such as aluminium ions, in the composition. These trivalent ions would otherwise combine with the carboxymethylcellulose binder to create a rubbery gel.

Sodium phosphate is added as a pH buffer. Methyl paraben and propyl paraben are both preservatives. Any of a variety of flavouring and colouring agents can be used.

The preferred level of sodium fluoride, 0.2244%, is the maximum allowed by the FDA in an over-the-counter fluoride toothpaste. This corresponds to 0.1% fluoride ion, with a little excess being allowed as an anticipated normal deviation. The given range for sodium fluoride, from 0.05 to 1%, corresponds to a maximum fluoride ion content of about 0.45%.

Naturally, the kaolin used must be of a relatively pure grade. It is well-known that excessive impurities in any abrasive can inhibit fluoride availability. Whiteness and brightness are often measures of purity, and it has been found desirable that the kaolin should have a brightness of at least 89 and a whiteness index of at least 13 or below.

Throughout this specification, the degree of calcination is referred to in terms of a percentage conversion to the mullite crystal form. However, reference to this crystal form is a convenient way of expressing the manner in which it can be determined whether a particular kaolin is or is not acceptable. In such a determination, no crystal structure studies have been made in an attempt to confirm that 80% of the particles had a particular crystal instructure. Each kaolin used has been studied by differential thermal analysis. In this study, uncalcined kaolin is heated gradually, in accordance with differential thermal analysis techniques, and it is found that, at about 975-990°C, an exothermic reaction occurs which gives off a significant amount of heat resulting in a peak on the analysis graph. On the understanding that this reaction is a result of the change in crystalline form from the gamma-alumina to the mullite form, it may be concluded from differential thermal analysis that, if a particular kaolin yields a peak at 975-990°C which is only 20% of the peak resulting from differential thermal analysis of an uncalcined kaolin, then the calcined kaolin under study is at least 80% mullite crystal in form.

Similarly, in discussing the gamma-alumina crystal form, this has not been based on crystal structure analysis. It has rather been based on the understanding that the significant endothermic reaction peak which occurs at about 520°C in a differential thermal analysis of uncalcined kaolin reflects the point at which the kaolin is converted to the gamma-alumina crystal form.

The following Examples (toothpastes A, B, C and D) illustrate the invention.

Examples 1 to 4

Four toothpastes were prepared using the particularly preferred formulation of Table I. The kaolins used were all more than 80% calcined to the mullite crystal form, respectively Satintone Special from Engelhard Minerals (toothpaste A), Al-Silate-O kaolin from Freeport (listed as Freeport kaolin) (B), Optiwhite from Burgess Pigments (C), and a kaolin with a larger average particle size than the Satintone Special (D).

Comparative Formulations

On the basis of the preferred formulation given in Table I, a toothpaste (J) was prepared using Kaolin GK1072 from Georgia kaolin. GK1072 is a gamma-alumina/mullite kaolin which was acquired from Indiana University, and is apparently that used in connection with the work which led to US-A-4,122,163. Toothpaste K was similarly made using Satintone Special before it was calcined to the normal extent. Toothpaste L uses Kaopolite SF which has the degree of calcination required in this invention, but a different particle size distribution.

Properties of the kaolins used in toothpastes A to D and J to L are given in Table II. The particle size ranges are given in microns.

TABLE II

| Kaolin | A | B | C | D | J | K | L |
|--------|-----|-----|-----|------|-----|-----|-------|
| 50.0-10.0 μ | 1% | 2% | 2% | 3.5% | 3% | 1% | 1% |
| 10.0-5.0 μ | 5% | 8% | 7% | 10% | 7% | 5% | 1% |
| 5.0-1.0 μ | 74% | 65% | 63% | | 61% | 74% | 33% |
| <1.0 μ | 21% | 25% | 28% | | 29% | 21% | 65% |
| % Calcination | 90-100 | 80-95 | 100 | > 80 | <50 | <50 | 80-95 |

Fluoride availability tests have been conducted on toothpastes A to D, J, K, L and two commercially available toothpastes respectively based on hydrated silica (toothpaste M) and calcium pyrophosphate (toothpaste N). The tests were conducted initially and after one, two and three months of accelerated aging. In each test formula, one milligram of theoretically available fluoride ion was provided for each gram of product. It is known that this is also the level of theoretically available fluoride in the two commercially available toothpastes, because this is the maximum theoretically available fluoride which is allowed, in the U.S.A., in an over-the-counter product. An initial fluoride availability of 0.96 means that 0.96 mg/g fluoride ion was actually available in water-soluble, ionised form. The initial fluoride availability of 1.065 for the commercially available hydrated silica fluoride toothpaste is the result of allowable variations from the maximum level of 1 mg/g.

Accelerated aging is achieved by storing the toothpaste at 40°C. It is generally accepted in the industry that one month of accelerated aging at 40°C is equivalent to eight months of ambient aging. An accelerated aging test was also performed for one month at 50°C,

8

10 C degrees in excess of the industry accepted standard. The results are given in Table III.

TABLE III

| Toothpaste | Initial Fluoride | Accelerated (months) | | | 1 month @ 50°C |
|---|---|---|---|---|---|
| | | 1 | 2 | 3 | |
| A | 0.965 | 0.96 | 0.94 | 0.92 | 0.97 |
| B | 0.96 | 0.94 | 0.89 | 0.92 | 0.935 |
| C | 0.945 | 0.915 | 0.875 | 0.895 | 0.89 |
| J | 0.975 | 0.875 | 0.895 | 0.865 | 0.895 |
| K | 0.915 | 0.87 | 0.80 | 0.80 | 0.79 |
| L | 0.875 | 0.84 | 0.765 | 0.78 | 0.83 |
| M | 1.065 | 1.04 | 1.055 | 0.995 | 1.02 |
| N | 0.90 | 0.87 | 0.805 | 0.82 | 0.865 |

While the fluoride toothpaste based on hydrated silica shows a high degree of fluoride availability even with aging, in vitro fluoride uptake tests on human enamel reveal that a toothpaste in accordance with the present invention is surprisingly superior to the hydrated silica toothpaste formulation. The tests are conducted, in accordance with OTC Panel Method No. 40, as follows:

1. 4 mm diameter enamel cylinders were excised from cleaned human incisors. The outer layer was removed by 2 M perchloric acid. The enamel was mounted and sealed with blue inlay casting wax. It was then ground flat with 600 grit silicon paper.

2. Each enamel piece was rotated in 2 M perchloric acid to remove 2 layers. One ml of acid was used for 30 seconds to obtain the first background layer, and then another one ml for 60 seconds to obtain the second. These solutions were analysed for fluoride by diluting them 1:1 v/v with alkaline Orion TISAB buffer. To obtain fluoride levels for the background enamel fluoride that are readable with the Orion Specific Ion Meter, the alkaline TISAB buffer used for dilution and buffering the perchloric acid etch solutions contains 0.2 ppm fluoride. This fluoride amount is subtracted out before data is used. Fluoride values were determined with an Orion Fluoride electrode.

3. The enamels were reground and decalcified for 24 hours with 0.025 M pH 4.5 lactate buffer modified with 0.0002 M disodium dihydrogen ethylidenehydroxydiphosphonate.

4. Each mounted enamel was rotated in supernatant from a 25% dentifrice slurry which was centrifuged for 30 minutes at 11,000 rpm.

5. After rinsing, two layers of enamel were removed with 2 M perchloric acid in the same manner as in the preteatment.

6. These enamel solutions were diluted 1:1 v/v with alkaline TISAB buffer, and the fluoride ion content was read with a fluoride electrode, as above.

7. After the background was subtracted, fluoride was reported as nanograms per enamel layer ± standard deviation. Results are given in Table IV.

TABLE IV

| Toothpaste | First Layer | Second Layer | Both Layers |
|---|---|---|---|
| A | 1760 ± 204 | 131 ± 24 | 1900 ± 187 |
| M | 530 ± 75 | 168 ± 43 | 700 ± 32 |
| N | 785 ± 89 | 90 ± 42 | 875 ± 88 |

The extent to which a toothpaste of the present invention reduces enamel solubility was also compared to the two commercially available toothpastes discussed above. The test procedure is based on OTC Method No. 33 and is as follows:

1. Tooth crowns of molars, cuspids and bicuspids were cleaned by polishing with pumice flour. Most of the root areas were removed. The crowns were mounted in epoxy cement F-88, six to a beaker, and sealed with blue inlay casting wax. Four sets of 6 crowns were used to test each dentifrice. Deprotection was accomplished at the beginning and between tests by 2 hours in 0.1 M pH 4.5 lactate buffer. This solution and all others were stirred at a speed close to 1725 rpm which is the speed for stirring as designated in OTC procedure No. 33.

2. Tests were carried out by determining the solution of the enamel in the buffer before and after the dentifrice treatment. The 0.1 M pH 4.5 lactate buffer was added to the beaker containing the 6 enamel crowns and stirred for 15 minutes at 37°C. The lactate was then removed and kept for the phosphate analysis. The crowns were rinsed 3 times at least.

3. 15 g of dentifrice were mixed with 45 ml of water and centrifuged. The supernatant was poured over the crowns and stirred for 5 minutes. Then the supernatant was discarded and the crowns rinsed well.

4. Again the lactate buffer was used on the crowns for 15 minutes, as before, in order to determine the solubility of the enamel after the treatment.

5. The solutions of lactate, pre- and post-treatment, were analysed for phosphate by the known Fiske and SubaRow method.

6. The percentage reduction in enamel solubility was calculated by subtracting the post-phosphate from the pre-phosphate, dividing by the pre-phosphate value and then multiplying by 100.

The average results for the three toothpastes are as given in Table V.

TABLE V

| Toothpaste | % Reduction in enamel solubility |
|---|---|
| A | 36.5 |
| M | 20 |
| N | 28 |

A toothpaste of the present invention is also superior to commercially available toothpastes in polishing and stain removal results. Excised human teeth (24 per treatment) were dulled with hydrochloric acid and initial reflectance measurements were made via spectro-radiometry techniques. The teeth were brushed in a brushing machine with the various toothpastes and reflectance measurements and spectro-radiometric measurements were made (before and after) to determine the level of polishing, as indicated by a change in photon radiance, and the level of stain removal as determined by spectro-radiometric comparison. Perceivable colour changes to the eye are also indicated in accordance with a mathematical index. These tests are in accordance with industry accepted standards. The results are given in Table VI.

TABLE VI

| Toothpaste | Mean Photon Radiance | Mean Stain Removal (%) | Perceivable Colour Change |
|---|---|---|---|
| A | 143.5 | 14.2 | 83.3 |
| D | 140.3 | 13.2 | 80.1 |
| M | 129.9 | 7.1 | 40.9 |
| N | 136.4 | 8.3 | 48.6 |

CLAIMS

1.     A fluoride toothpaste characterised in that it contains calcined kaolin of which at least 80% by weight is calcined to the mullite crystal form, in which the majority of the particles are sufficiently fine to afford good polishing without excess abrasion, but no more than 35% by weight of the particles are less than 1 micron in size.

2.     A fluoride toothpaste according to claim 1, wherein more than 50% by weight of the calcined kaolin particles are between 1 to 10 microns, and less than 5% by weight of the particles exceed 10 microns, in size.

3.     A fluoride toothpaste according to claim 2, wherein more than 50% by weight of the particles are from 1 to 5 microns, and less than 10% by weight are from 5 to 10 microns, in size.

4.     A fluoride toothpaste according to claim 3, wherein less than 1% by weight of the particles are from 10 to 50 microns, less than 5% by weight of the particles are from 10 to 5 microns, at least 70% by weight of the particles are from 1 to 5 microns, and no more than 30% by weight of the particles are less than 1 micron, in size.

5.     A fluoride toothpaste according to any preceding claim, wherein at least 90% by weight of the kaolin is calcined to the mullite crystalline form.

6.     A fluoride toothpaste according to any preceding claim, wherein the active fluoride ingredient is sodium fluoride.

7.     A fluoride toothpaste according to any preceding claim, which comprises from 5 to 70% by weight of the calcined kaolin, from 0.02 to 0.5% by weight water-soluble fluoride ion; from 5 to 70% by weight humectant; and from 0.5 to 10% by weight binder.

8.     A fluoride toothpaste according to claim 7, which additionally comprises from 0.5 to 5% by weight surfactant.

9.     A fluoride toothpaste according to claim 8, which comprises from 35 to 40% by weight of the calcined kaolin.

10.     A fluoride toothpaste according to any preceding claim, which comprises from 0.05 to 10% by weight titanium dioxide whitener.